(19) Europäisches Patentamt
European Patent Office
Office européen des brevets

(11) EP 0 729 944 B1

(12) **EUROPEAN PATENT SPECIFICATION**

(45) Date of publication and mention of the grant of the patent:
**05.06.2002 Bulletin 2002/23**

(51) Int Cl.[7]: **C07D 201/08**

(21) Application number: **96200500.5**

(22) Date of filing: **27.02.1996**

(54) **Process for the preparation of epsilon-caprolactam**

Verfahren zur Herstellung von Epsilon-Caprolactam

Procédé pour la préparation d'epsilon-caprolactame

(84) Designated Contracting States:
**BE DE ES FR GB IT NL**

(30) Priority: **01.03.1995 US 396240**

(43) Date of publication of application:
**04.09.1996 Bulletin 1996/36**

(73) Proprietors:
- **DSM N.V.**
  **6411 TE Heerlen (NL)**
- **E.I. DU PONT DE NEMOURS AND COMPANY**
  **Wilmington Delaware 19898 (US)**

(72) Inventors:
- **Buijs, Wim**
  **6365 BM Schinnen (NL)**
- **Wolters, Henricus Franciscus Wilhelmus**
  **6101 BT Echt (NL)**

(74) Representative: **Kleiborn, Paul Erik et al**
**DSM Patents & Trademarks**
**Office Geleen**
**P.O. Box 9**
**6160 MA Geleen (NL)**

(56) References cited:

| | |
|---|---|
| **EP-A- 0 376 123** | **DE-A- 3 602 375** |
| **DE-A- 3 602 376** | **DE-A- 3 602 377** |

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 729 944 B1

## Description

The invention relates to a process for the preparation of ε-caprolactam starting from a 5-formylvaleric acid or ester or amide (aldehyde compound) in which a reaction in the presence of ammonia and hydrogen and a subsequent cyclization of the reaction products thus formed (ε-caprolactam precursors) to ε-caprolactam is performed in the presence of water.

Such a process is known starting from methyl 5-formylvalerate and is described in US-A-4730040. In this process methyl 5-formylvalerate is first (x) hydrolyzed in the presence of water and an acidic agent to 5-formylvaleric acid, which is then further (y) reacted in a water solvent with ammonia and hydrogen in the presence of a Raney nickel catalyst in one reaction step to 6-aminocaproic acid. After separation of all the ammonia the aqueous mixture is heated to 300°C (z) and ε-caprolactam is formed by cyclization of the 6-aminocaproic acid.

A disadvantage of this process is that, according to the experimental results, the best yield of step (x) is only 78%, the best yield of step (y) is only 77% and the best yield of the final reaction (z) to ε-caprolactam is only 95%. The overall yield is thus at most 57%. This overall yield to ε-caprolactam is too low for a commercially attractive process.

The object of the present invention is a process for preparing ε-caprolactam in a high yield starting from 5-formylvaleric acid, ester or amide.

The object of the invention is arrived at in that the following steps are performed:

(a) contacting the 5-formylvalerate acid, ester or amide with ammonia and water in the absence of hydrogen and/or a hydrogenation catalyst,

(b) contacting the resulting aqueous mixture of step (a) with hydrogen in the presence of ammonia and a hydrogenation catalyst, in which the water content is higher than 10 wt.%,

(c) heating the resulting mixture of step (b) to a temperature between 200 and 350°C in order to convert the reaction products of step (b) to ε-caprolactam.

It appears that when the process is performed in the above described fashion high yields to ε-caprolactam are obtained starting from the 5-formylvaleric acid, ester or amide. A further advantage of this process is that the solvent of the process is water. Water is a reaction product of the reactions taking place in the process according to the invention. By using additional water as a solvent the number of different compounds in the process is limited. Water is furthermore easy to handle in large scale commercial plants. Less safety measures are needed when water is used as solvent because of its, for example, non-toxic, non-explosive and non-carcinogenic properties. Another advantage is that the same solvent is used in the whole process making it possible to use the reaction mixture obtained in one step without undue burden in the next step.

A further advantage is that high yields to ε-caprolactam are possible when applying a relatively high concentration (concentration levels higher than 10 wt.%) of starting or intermediate compounds in the various steps (a)-(c). This is advantageous because lower-volume process equipment is needed, thus reducing the costs of investment for a commercial process.

A similar process starting from a 5-formylvalerate ester to ε-caprolactam is described in JP-B-68029148. This patent publication describes a one step proces in which a 5-formylvalerate is reacted with ammonia and hydrogen in the presence of water at 230°C and 15 MPa in the presence of Raney nickel in the liquid phase. A disadvantage of this process, according to US-A-4730041, is that the yields fluctuate very greatly when the process is carried out industrially.

A process for the cyclization of 6-aminocaproic acid or 6-aminocaproic acid amide (intermediate products of the present invention which are formed in step (b)) in an aqueous ammonia or in pure water solvent is described in US-A-3485821. Experimental results show that at 33 wt.% 6-aminocaproic acid in water and 300°C polyamide is formed as a by-product.

In an article of Frank Mares and Desmond Sheehan (Ind. Eng. Chem. Process Des. Dev. Vol. 17, No. 1, 1978, page 9-16) the cyclization of 6-aminocaproic acid, ester and amide in either a water solvent or an ethanol solvent is described. This article teaches that a temperature of about 300°C and at higher concentrations of 1 mol/kg of 6-aminocaproic acid or 6-aminocaproic acid amide in water high levels of dimers and other oligomers may be formed. According to this article the formation of these oligomers is disadvantageous for the ε-caprolactam yield.

The possible starting compounds of the present process, 5-formylvalerate ester, 5-formylvaleric acid and 5-formylvaleric acid amide will be referred to as the aldehyde compound in the description if the description is applicable to all three starting compounds or mixtures of these compounds.

The mixture of compounds obtained in step (b) will contain reaction products which are able to react to ε-caprolactam and possibly some amount of ε-caprolactam precursors. These "ε-caprolactam" precursors are 6-aminocaproate ester, 6-aminocaproic acid and 6-aminocaproic acid amide. The amount of 6-aminocaproate ester will normally be very low. When relatively high concentrations of aldehyde compound is applied in step (a) and (b) a significant

amount of oligomers (mostly dimers) of the foregoing compounds are also formed. It has been surprisingly found that these oligomers can be reacted to ε-caprolactam in step (c) with similar high yields compared to when starting from the three ε-caprolactam precursors listed above. Because of this the oligomers formed in the process according to the invention are also considered to be ε-caprolactam precursors.

The 5-formylvalerate ester, acid or amide can be obtained by hydroformylation of the corresponding pentenoate ester, acid or amide as for example described for the ester in WO-A-9426688 and WO-A-9518089 and for the acid in WO-A-9518783. Preference is given to the 5-formylvalerate ester as starting compound because this compound is more easily obtainable.

The aldehyde compound can be represented by the following general formula:

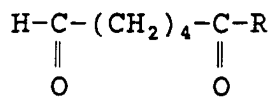

(1)

where R is a -OH, $-NH_2$ or $-O-R^1$ group, $R^1$ is preferably an organic group with 1 to 20 carbon atoms, wherein the organic group is an alkyl, cycloalkyl, aryl or aralkyl group. More preferably $R^1$ is an alkyl group. Examples of $R^1$ groups include methyl, ethyl, propyl, isopropyl, n-butyl, tert-butyl, isobutyl, cyclohexyl, benzyl and phenyl. By preference $R^1$ is methyl or ethyl.

The first step (a) is carried out under non-hydrogenating conditions. The term 'non-hydrogenating conditions' means that the reaction conditions are such that, either no hydrogen is present or if hydrogen is present, then the aldehyde compound or a reaction product of it is not or virtually not reduced by the hydrogen. In general, non-hydrogenating conditions are realized by carrying out the first step in the absence of a hydrogenation catalyst.

In one such embodiment of the present process the hydrogen (of step (b)) can be already present in step (a). However, if the hydrogenation catalyst is already present in this step, non-hydrogenating conditions are achieved by not adding hydrogen to the reaction mixture until after completing step (a). A third possible embodiment is that neither hydrogen nor the hydrogenation catalyst is present in step (a).

The temperature in step (a) may be up to 120°C and is preferably between 0°C and 100°C. More preferably the temperature is between 20-100°C.

It has been found that the best results with regard to yield to ε-caprolactam precursors are achieved when the conversion of the aldehyde compound in step (a) is more than 90%, preferably more than 99%. Too low of a conversion will result in an increase of, for example, the 6-hydroxycaproate ester (or acid or amide) and/or secondary amines formation. Formation of these compounds will negatively influence the overal yield to ε-caprolactam.

As explained above a too low contact or residence time in step (a) will result in undesirable by-product formation. The optimal residence or contact time at which the conversion of the aldehyde starting compound is virtually completed will depend on the reaction conditions, for example temperature, concentration of reactants and method of mixing. Longer contact or residence times than needed to achieve the above conversion are possible. The optimal residence time or contact time can be easily determined by the man skilled in the art. Starting from the temperature and concentration range here described the residence or contact time will under normal mixing condition preferably be higher than 5 seconds. Preferably the residence or contact time will be below 2 minutes.

Step (a) is carried out in the presence of ammonia, preferably a molar excess of ammonia is chosen such that the molar ratio ammonia:aldehyde compound is between 1:1 and 500:1 calculated on the starting amount of the aldehyde compound. Preferably this ratio is higher than 5:1. More preferably this ratio is higher than 10:1. If this ratio is too low the ε-caprolactam yield is negatively influenced.

Water will be formed in step (a) as a reaction product of the reaction between the aldehyde compound and ammonia. The amount of water in the starting mixture of step (a) and the water formed in step (a) is preferably sufficient to amount to at least the 10 weight % water concentration of the mixture used in step (b). More preferably step (a) is performed in the presence of at least 10 weight percent of water.

When the aldehyde starting compound is a 5-formylvalerate ester preferably an alkanol is present in step (a). The alkanol is preferably the corresponding alcohol of the ester ($R^1$-OH). The concentration of alkanol in step (a) is preferably between 2 and 20 and more preferably between 5 and 15 wt.%. When an alkanol is present in step (a) the solubility of the 5-formylvalerate ester in the reaction medium increases.

Preferably the molar ratio ammonia:5-formylvalerate (5-formylvalerate plus ε-caprolactam and/or ε-caprolactam precursors) in step (a) is between 3:1 and 25:1 more preferably between 5:1 and 15:1.

The water content of the reaction mixture in step (a) is preferably between 15-60 wt.% and more preferably between 20-50 wt.%.

The concentration of the aldehyde compound or the concentration of the sum of aldehyde compound and its reaction products (5-formylvalerate plus ε-caprolactam and/or ε-caprolactam precursors) in step (a) is generally between 1 and 50 wt.% and preferably between 10 and 35 wt.%.

The pressure in the first step is not critical. The pressure is generally equal or greater than the resulting equilibrium pressure of the reaction mixture and the temperature employed.

Step (a) can be carried in the presence of a catalyst, for example an acid ion exchanger or an acidic metal oxide catalyst, for example alumina or $TiO_2$. The conversion of the aldehyde starting compound in the first step also proceeds favorably in the absence of a catalyst. Because the overall yield to ε-caprolactam is not greatly influenced by the presence of a catalyst in the first step, such a catalyst is generally not used.

The process according to the invention can be performed batch wise or continuously. A large scale commercial process will preferably be performed continuously. For step (a), it is important that the reactants are sufficiently contacted at a certain temperature during a specified period of time optionally in the presence of a catalyst as described above. Any manner of contacting will usually suffice. For example a tube reactor with or without, for example, internal baffling or packing or a static mixer is a possible contacting unit for step (a). To control the temperature in step (a) it may be advantageous to use cooling devices, for example cooled walls or a cooling spiral placed in the contacting unit.

The above described ratios and concentrations and their preferred values for step (a) also apply for step (b) unless otherwise mentioned. Moreover the composition of the aqueous reaction mixture obtained in step (a) is by preference, directly and without substantial separation of any of the compounds of the mixture, used in step (b). This is advantageous because it results in a more simple process.

The reaction product obtained in step (a) is converted in step (b), to ε-caprolactam and ε-caprolactam precursors, under hydrogenating conditions in the presence of ammonia.

The ε-caprolactam precursors, 6-aminocaproic acid amide, 6-aminocaproate ester or 6-aminocaproic acid, are defined by the following formula:

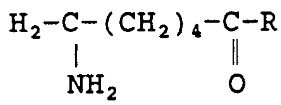

(2)

where R is the same as in formula (1). The oligomers are for the greater part dimers of 6-aminocaproic acid or dimers of 6-aminocaproic acid amide. Trimers and higher oligomers may also be formed.

When the aldehyde compound is 5-formylvalerate ester a mixture of ε-caprolactam, 6-aminocaproic acid, 6-aminocaproic amide and none or a small amount of 6-aminocaproate ester and/or oligomers and the corresponding alcohol will be obtained in step (b). The hydrolysis of the ester-group mainly takes place in step (b). When the aldehyde compound is 5-formylvaleric acid a mixture of ε-caprolactam, 6-aminocaproic acid and possibly some 6-aminocaproic acid amide and possibly some oligomers will be obtained in step (b). Starting from 5-formylvaleric acid amide the main product will be 6-aminocaproic acid amide and possibly some oligomers next to some ε-caprolactam.

By 'hydrogenating conditions' is understood in this invention that the reaction conditions are such that the intermediate reaction product(s) obtained in step (a) can be reduced by hydrogen. In general hydrogenation conditions are achieved when hydrogen and a hydrogenation catalyst are present.

The total pressure used in step (b) is generally between 0.5 and 20 MPa. The pressure is preferably between 0.5-10 MPa and more preferably between 1.0-5.0 MPa.

Step (b) is in general carried out at a temperature higher than 40°C. In general the temperature will be lower than 200°C. To achieve optimal overall yields to ε-caprolactam the temperature is more preferably between 70 and 180°C and most preferably between 80 and 160°C.

The residence or contact time in step (b) should be high enough to reduce virtually all the intermediate products formed in step (a) to ε-caprolactam and ε-caprolactam precursors. Higher residence time or contact time will result in that more ε-caprolactam and possibly oligomers are formed. The residence or contact time is preferably between around a half minute to a couple of hours. When the process is carried out batch wise or in a continuously operated slurry reactor the contact or residence time respectively will generally be higher than the residence time when a continuously operated tube reactor is used.

The hydrogenation catalyst comprises one or more of the metals choosen from the metals of groups 8-10 of the Periodic System of the Elements (Handbook of Chemistry and Physics, 70th edition, CRC Press, 1989-1990) for example nickel, cobalt, ruthenium, platinum or palladium. Preference is given to Ru-, Ni- or Co-containing catalysts. In addition to Ru, Co and/or Ni the catalysts can also contain other metals for example Cu, Fe and/or Cr. The content of these additional metals may be, for example, up to 20% by weight based on the total metal content. The catalytically

active metals may be applied onto a carrier or not. Suitable carriers are for example aluminium oxide, silica, titanium oxide, zirconium oxide, magnesium oxide and carbon. Non-carried metals can also be used. An example of a non-carried metal is finely dispersed ruthenium. Preferred Ni- and Co-containing catalysts are Raney nickel and Raney cobalt optionally in combination with small amounts of another metal, for example Cu, Fe and/or Cr. Most preferred are ruthenium containing catalysts. High yields to ε-caprolactam over a prolonged period of time are possible when using this class of catalysts. Examples of possible ruthenium catalysts are non-carried metals, for example finely dispersed ruthenium or ruthenium on a carrier, for example a carbon or alumina carrier.

Step (b) may be performed continuously in a fixed bed reactor in which the heterogeneous hydrogenation catalyst is present. An advantage of this reactor is that the reactants are easily separated from the hydrogenation catalyst. Another manner of operating step (b) is by way of one or more continuously operated well mixed contactors in series in which the hydrogenation catalyst is present as a slurry (slurry reactor). This manner of operation has the advantage that the heat of the reaction of step (b) can be easily controlled by, for example, a cooled feed or by way of internally placed cooling devices. Examples of specific and suitable slurry reactors are one or multiple staged bubble columns or a gas lift-loop reactor or a continuously stirred tank reactor (CSTR). The slurry-hydrogenation catalyst can be separated from the reaction mixture after step (b) by for example using hydrocyclones and/or by filtration, for example by cake- or cross-flow filtration.

The catalyst concentration in step (b) may be choosen between a wide range. In a fixed bed reactor the amount of active metal per volume will be high while in a slurry reactor this concentration will in general be lower. In a continuously operated slurry reactor the weight fraction of catalyst (including the carrier) is typically between 0.1 and 30 weight % relative to the total content of the reactor. The weight fraction will for example depend on the use of a carrier and the kind of carrier.

Ammonia, hydrogen, the hydrogenation catalyst and the alkanol (if present) are preferably separated from the reaction mixture obtained in step (b) prior to step (c). Hydrogen and part of the ammonia can be advantageously be separated by reducing the pressure and performing a gas/liquid separation. An example of such an operation is a flash operation performed at between ambient pressure and 0.5 MPa. The hydrogen and ammonia can advantageously be recycled to steps (a) and (b).

In a subsequent step the alkanol can be separated. It has been found that it is advantageous to perform the cyclization step (c) in the pressence of less than 1 wt% and more preferably less than 0.1 wt% of alkanol. Thus when the resulting mixture of step (b) contains alkanol it is advantageous to separate this compound. It has been found that the pressence of alkanol during the cyclization promotes the formation of the corresponding N-alkyl caprolactam, an unwanted by-product. The presence of small quantities of these N-alkylated products, for example N-methyl ε-caprolactam, in final ε-caprolactam makes the ε-caprolactam less suitable for use as starting material for nylon-6 fibers. Because these N-alkylated products are difficult to separate from the final ε-caprolactam it is favorable that they do not form or that their formation is minimized in the process according to the invention.

Separating the alkanol may be performed by any known method known to the man skilled in the art, for example distillation or stripping, for example steam stripping.

Preferably the alcohol is removed by stripping the aqueous mixture with steam. In a commercial large scale process the stripping preferably involves the continuous counter current contacting of the aqueous starting mixture with upflowing steam in a vertical positioned column, in which at the top a water/alcohol stream and at the bottom an alcohol-poor aqueous product stream is obtained. Steam stripping is advantageous because the alcohols can be removed very effectively and because a convenient concentration of the ε-caprolactam precursors and ε-caprolactam in resulting aqueous mixture is obtained. In this process ammonia is also quantitatively removed. The water/alcohol/ammonia thus obtained can be advantageously recycled to step (a).

The steam stripping is preferably performed at a pressure between ambient pressure and 1.0 MPa, and more preferably under near atmospheric conditions. Near atmospheric conditions are preferred because less expensive process equipment is required.

The concentration of ammonia in step (c) is preferably below 5 wt.% and more preferably below 3 wt.%. High concentrations of ammonia have a negative effect on the yield to ε-caprolactam (per pass).

The concentration of ε-caprolactam precursors in step (c) is preferably between 5 on 50 wt.% and more preferably between 10-35 wt.%. and most preferred above 15 wt.%.

The temperature in step (c) is between 200 and 350°C. Preferably the temperature is between 270 and 330°C. More preferably the temperature in step (c) is higher than 285°C in step (c), because higher selectivities to ε-caprolactam and thus a higher overall yield to ε-caprolactam is obtained. Temperatures exceeding 330°C are less preferred because of the increasing formation of unwanted by-products.

The pressure in step (c) is preferably between 5.0 and 20 MPa. Normally this pressure will be greater than or equal to the resulting pressure of the reaction mixture and the temperature employed.

Step (c) can be performed continuously in process equipment resulting in high and low rates of backmixing, for example in a (optionally a series of) continuously stirred tank reactor(s) (CSTR) or a tube reactor.

The ε-caprolactam can be separated from the reaction mixture obtained in step (c) by for example crystallization, extraction or by distillation. Examples of possible extraction agents are methylene chloride, cyclohexane, toluene, benzene, chloroform or trichloroethane.

After separating the ε-caprolactam from the mixture obtained in step (c), the resulting mixture, containing unconverted ε-caprolactam precursors comprising also some oligomers is preferably recirculated to step (c). Preferably not all of the ε-caprolactam is separated from the mixture obtained in step (c) if the ε-caprolactam is separated by distillation. It has been found that the oligomers are more easily handled when the distillation residue mixed with some ε-caprolactam, preferably between 5 and 50 wt.% ε-caprolactam. By performing step (c) in this way it has been found that almost no build-up of oligomers in the circulating mixture takes place and that the overall yield to ε-caprolactam of the cyclization step (c) of practically 100% is possible.

An example of a possible embodiment of the process starting from methyl 5-formylvalerate according to the invention is given in Figure 1. The illustrated process is a schematic representation of the process equipment used in the below examples.

In figure 1 a mixture of methyl 5-formylvalerate/water/ammonia/methanol (1) is led to step (a). Step (a) is performed in a tube reactor (A). The resulting aqueous reaction mixture (2) is fed to step (b), performed in a continuously stirred tank reactor (B), in which a catalyst is present as a slurry. Hydrogen (3) is continuously or intermittently fed to step (b) in which the desired hydrogen partial pressure is maintained at a constant value. From the aqueous reaction mixture obtained in step (b) the slurry catalyst is separated by filtration (not shown). Hydrogen and part of the ammonia are separated from the mixture (4) by flashing in flasher (F). Methanol and the residual ammonia are separated from (6) in a steam stripper (S) in which steam is supplied to via (8). The resulting mixture (9) is fed to step (c) together with recycle (14) and fresh water (15). Step (c) is performed in a tube reactor (C). From (10) first water is separated in distillation unit (D1). From the mixture (12) ε-caprolactam (13) is separated in a second distillation unit (D2). The residue contains ε-caprolactam + ε-caprolactam precursors, comprising also some oligomers. This mixture (14) is recirculated to step (c). In the recirculating streams purges will be present (not shown) to overcome possible build up of contaminants and by-products.

The invention will be elucidated with the following examples.

The composition of the resulting mixtures of the experiments are sometimes expressed in mol percentages. The molar percentage of a component is represented by the molar fraction (* 100%) of the molar amount of converted methyl 5-formylvalerate (M5FV) which contributes to that specific component. For example if the starting amount of M5FV is 100 mol and the resulting mixture contains 50 mol ε-caprolactam and 25 mol dimers then the molar contribution to ε-caprolactam will be 50 mol% and the molar contribution to the dimers will be 50 mol% (totaling 100 mol%). When no oligomers such as dimers are present in the mixture the above molar percentages are the same as the molar yield as expressed below:

$$\text{yield of component x} = \frac{\text{mol component x formed}}{\text{mol M5FV converted}} * 100\%$$

<u>Example I</u>

At a pressure of 5.0 MPa, 45 g/hr (312 mmol/hr) of methyl 5-formylvalerate, 495 g/hr (27.5 mol/hr) of water and 360 g/hr (21 mol/hr) of ammonia was pumped through a tube which was cooled by a water bath so that a constant temperature of 35°C was maintained in the tube. Almost no back mixing occurred and the (liquid) residence time was 15 seconds. The reaction mixture leaving the tube did not contain any significant amount of methyl 5-formylvalerate. The resulting mixture leaving the tube (first step) was fed to a continuously stirred tank reactor, a Hastelloy C autoclave of 1 liter liquid volume. The reactor was stirred at 1250 rpm. The pressure was kept constant at 5 MPa. The residence time was 60 minutes and the temperature was kept at 100°C. To the reactor a net amount of 10 g/hr of hydrogen was fed. The reactor was filled with 50 g of an unpromoted Raney Nickel catalyst (93 wt.% nickel and 7 wt.% aluminum, average particle size: 59 µm from Activated Metals Company (A5000)). The rate of the effluent was such that the level of the liquid in the reactor was kept at a constant height.

The effluent was analyzed by High Pressure Liquid Chromatography (HPLC) after 3 and 6 hours of operation. The results are summarized in Table 1. The yields after 3 and 6 hours of operation were comparable and within their respective error range. The conversion of methyl 5-formylvalerate was 100%.

TABLE 1

| | 3 hours | 6 hours |
|---|---|---|
| 6-aminocaproic acid amide | 58.7 (1) | 59.8 |
| 6-aminocaproic acid | 23.9 | 25.3 |
| methyl-6-aminocaproate | 0.4 | 0.0 |
| ε-caprolactam | 17.0 | 14.9 |

(1) Results in weight percentage.

As is clear from Table 1 high yields, up to a total of 100%, are obtained of ε-caprolactam and ε-caprolactam precursors when using water as solvent.

Example II

Example I was repeated using 39.9 g/hr of methyl 5-formylvalerate, 184 g/hr ammonia and 644 g/hr of water. The residence time in step (a) was 15 seconds and in step (b) 60 minutes. The catalyst concentration was 60 g/liter and the liquid level was kept constant maintaining a liquid volume in the reactor of step (b) of 1 l.

After step (b) a gas/liquid separation at reaction conditions the liquid reaction mixture thus obtained contained no starting methyl 5-formylvalerate (100% conversion), 7.2 g/h methanol, 170 g/hr ammonia, 647 g/hr water, 12.6 g/hr 6-aminocaproic acid, 12 g/hr 6-aminocaproic acid amide, 4.3 g/hr ε-caprolactam.

The selectivity to ε-caprolactam and ε-caprolactam precursors of step (a) + (b) was 81.9%.

Example III

Example II was repeated using 38.7 g/hr methyl 5-formylvalerate, 299 g /hr ammonia and 407 g/hr water.

After the gas/liquid separation at reaction conditions the liquid stream had the following composition: 8.5 g/hr methanol, 275 g/hr ammonia, 410 g/hr water, 8.7 g/hr 6-aminocaproic acid, 20.7 g/hr 6-aminocaproic acid amide and 4.8 g/hr ε-caprolactam,

The conversion of methyl 5-formylvalerate ester was 100% and the selectivity of step (a) and (b) was 99.7%.

Example II and III illustrate the influence of the ammonia-water molar ratio on the selectivity to ε-caprolactam and ε-caprolactam precursors.

Example IV-V

Example II was repeated at 2 MPa, with a catalyst concentration of 50 g/l with different feed compositions. The results are in Table 2:

TABLE 2:

| Example nr. | M5FV g/h | $NH_3$ g/h | $H_2O$ g/h | Selectivity % |
|---|---|---|---|---|
| IV | 42.6 | 93.3 | 770 | 93.4 |
| V | 40.4 | 173.6 | 639 | 97.5 |

Example VI-IX

Example II was repeated at different hydrogen pressures with a catalyst concentration of 50 g/l. The results are in Table 3.

TABLE 3:

| Example nr. | Pressure MPa | M5FV g/h | $NH_3$ g/h | $H_2O$ g/h | selectivity |
|---|---|---|---|---|---|
| VI | 1.6 | 42.3 | 92.5 | 766 | 94.4 |
| VII | 2.0 | 42.6 | 93.3 | 770 | 93.4 |
| VIII | 3.0 | 42.4 | 93.8 | 761 | 90.8 |

TABLE 3: (continued)

| Example nr. | Pressure MPa | M5FV g/h | $NH_3$ g/h | $H_2O$ g/h | selectivity |
|---|---|---|---|---|---|
| IX | 4.0 | 42.4 | 91.7 | 754 | 85.9 |

Examples I-IX illustrate that high selectivities and yields to ε-caprolactam and ε-caprolactam precursors can be achieved by varying for example the hydrogen pressure or the water-ammonia ratio.

Example X

Example I was repeated for 50 hours at a pressure of 3 MPa, in which 81.7 g/hr of M5FV, 203 g/hr ammonia and 526 g/hr water was pumped through the tube at a temperature of 35°C. Almost no back-mixing ocurred in the tube and the liquid residence time in the tube was 15 seconds. The reaction mixture leaving the tube contained no M5FV.

The mixture leaving the tube was fed to the continuously stirred tank reactor (CSTR) in which a liquid hold-up of 1 liter of liquid was maintained. The catalyst in the CSTR was a 5wt% ruthenium on $Al_2O_3$ catalyst (Engelhard: ESCAT 44) and the catalyst concentration was maintained at 103 g/l. The CSTR was stirred at 1260 rpm. The pressure in the CSTR was kept constant at 3 MPa and the temperature at 120°C. The residence time was 60 minutes. To the reactor a net amount of 5.0 g/hr of hydrogen was fed.

The effluent of the CSTR was analyzed by HPLC every 4 hours. The composition of the effluent did not vary significantly during the 50 hours of operation. The average composition of the effluent in the last 28 hours was 28 mol% 6-aminocaproic acid (6ACA), 47.2 mol% 6-aminocaproic acid amide (6ACAM), 24.2 mol% ε-caprolactam (CAP), 0.6 mol% methyl 6-aminocaproate (M6AC). No detectable amount of N-methyl caprolactam was present in this mixture. Thus a 100 mol% yield to ε-caprolactam and ε-caprolactam precursors is obtained in step 1 and 2.

Example XI

Example X was repeated for 22 hours, in which the water feed was replaced by a mixture of water and methanol (15 wt% methanol). The feed rate of this mixture was 511 g/hr. Almost no back-mixing ocurred in the tube and the liquid residence time in the tube was 15 seconds. The reaction mixture leaving the tube contained practically no methyl-5-formylvalerate.

The catalyst concentration in the CSTR was 96.0 g/l.

The composition of the effluent did not vary significantly during the 22 hours of operation. The average composition of all the products formed in the last 12 hours was 22.5 mol% 6ACA, 48.0 mol% 6ACAM, 27.4 mol% ε-caprolactam, 2.1 mol% methyl 6-aminocaproate. No detectable amount of N-methyl caprolactam was present in this mixture. Thus a 100 mol% yield to ε-caprolactam and ε-caprolactam precursors is obtained in step 1 and 2 when methanol is present in the feed to step 1.

Example XII

Example XI was repeated for 34 hours at a pressure of 3 MPa, in which 154 g/hr of methyl-5-formylvalerate, 205 g/hr ammonia and 439 g/hr of the 15wt%-methanol/water mixture was pumped through the tube at a temperature of 35°C. Almost no back-mixing ocurred in the tube and the liquid residence time was 15 seconds. The reaction mixture leaving the tube contained no methyl-5-formylvalerate.

The catalyst concentration in the CSTR was 209 g/l and the net hydrogen feed to the CSTR was 10 g/h. The temperature and pressure were the same as in Examples X and XI. The residence time was 60 minutes.

The composition of the effluent did not vary significantly during the 34 hours of operation. The composition of all the products present in the effluent of the CSTR after 34 hours was 19.6 mol% 6ACA, 36.9 mol% 6ACAM, 31.5 mol% ε-caprolactam, 2.4 mol% methyl 6-aminocaproate and 9.6 mol% oligomers. Most of the oligomers (about 90 wt% of the oligomers) were dimers of either 6ACA or 6ACAM. No N-methyl caprolactam was detected in this mixture. The yield to ε-caprolactam and ε-caprolactam precursors is 90.4 mol%. If all of the oligomers contribute to the final ε-caprolactam yield (after the cyclization step (c)) the yield of step (a) and (b) is practically 100 mol%.

This example illustrates that a high selectivity to ε-caprolactam and ε-caprolactam precursors is possible at a relatively high initial substrate concentration of 19.3 wt% M5FV.

Example XIII

Example XI was repeated for 22 hours at a pressure of 3 MPa, in which 192 g/hr of M5FV, 237 g/hr ammonia

and 357 g/hr of the 15 wt%-methanol/water mixture was pumped through the tube at a temperature of 35°C. Almost no back-mixing ocurred in the tube and the liquid residence time was 15 seconds. The reaction mixture leaving the tube contained no M5FV.

The catalyst concentration in the CSTR was 209 g/l and the net hydrogen feed to the CSTR was 10 g/h. The temperature and pressure were the same as in Examples X and XI. The residence time was 60 minutes.

The mixture leaving the CSTR was mixed with 114 g/hr of water after which the pressure was reduced to ambient pressure in a flash operation. The additional water was added before the flash operation and provided additional cooling. The composition of the effluent did not vary significantly during the 22 hours of operation. The composition of all the products present in the effluent of the CSTR at 22 hours was 14 mol% 6ACA, 40 mol% 6ACAM, 34 mol% ε-caprolactam, 0 mol% methyl 6-aminocaproate and 12 mol% oligomers. Most of the oligomers (about 88 wt% of the oligomers) were dimers of either 6ACA or 6ACAM. No detectable amounts of N-methyl caprolactam ware present in this mixture. The yield to ε-caprolactam and ε-caprolactam precursors is 88 %. If all of the oligomers contribute to the overal ε-caprolactam yield, then the yield of the combined step (a) and (b) is 100 mol%.

This example illustrates that a 100 % yield to ε-caprolactam and ε-caprolactam precursors is possible at a relatively high initial substrate concentration of 24.4 wt% M5FV.

Example XIV

The effluent of Example XII was during 34 hours continuously flashed at 0.11 MPa. Also 100 gr/hr $H_2O$ was continuously fed to this flasher to provide additional cooling. The resulting liquid stream, after flashing, had a rate of 612 gr/hr and contained 4.8 wt% $NH_3$, 6.5 wt% methanol, 66.0 wt% $H_2O$ and a total of 21.7 wt% of ε-caprolactam and ε-caprolactam precursors (1.07 mol/hr). After 34 hours 20.8 kg (36.4 mol ε-caprolactam or ε-caprolactam precursors) of this mixture was collected.

The collected mixture was subsequently continuously fed to a cyclization reactor at a rate of 500 gr/hr and at a temperature of 300°C. The cyclization was carried out in a plugflow reactor (almost no back-mixing) at a constant temperature of 300°C (maintained with the use of an oil bath), a pressure of 10 MPa and at a residence time of 30 minutes. The effluent leaving the cyclization reactor was cooled down and depressurized to ambient conditions. The average composition of all the products present in the liquid aqueous stream amounted to 65.9 mol% CAP, 5.1 mol% N-methyl caprolactam, 3.6 mol% 6ACA, 7.2 mol% 6ACAM and 18.2 mol% oligomers.

By vacuum distillation $H_2O$, $NH_3$ and methanol were semicontinuously removed from this liquid aqueous mixture. From the bottom stream of the first distillation 2515 gr ε-caprolactam (22.26 mol) and 234 gr N-methyl caprolactam (1.84 mol) were recovered by a second vacuum distillation. In the second distillation 1464 gr residue (bottom product) was obtained, which according to the mass-balance contained 12.3 mol equivalent monomeric products. Analysis of the residue showed that CAP, 6ACA, 6ACAM and oligomers were present.

This residue together with fresh water was fed continuously to the same cyclization reactor as described above. The rate of the residue was 100 gr/hr and the rate of the fresh water was 400 gr/hr. Cyclization of the residue was carried out at 300°C, 10 MPa and at a residence time of approximately 30 minutes. After cooling and depressurizing of the effluent of the cyclization reactor the average composition of all the products present in the liquid aqueous stream amounted to 71.7 mol% ε-caprolactam, 10.9 mol% of 6ACA and 6ACAM and 17.4 mol% oligomers. No detectable amounts of N-methyl caprolactam were present in the product mixture.

With two vacuum distillations as described above 924 gr ε-caprolactam (8.18 mol) was recovered from the liquid aqueous stream. Also 489 gr of residue was obtained in the second distillation, which according to the mass-balance should contain 4.11 mol caprolactam and/or caprolactam precursors.

The residue and fresh water was fed continuously to the same cyclization reactor as described above. The rate of the residue was 100 gr/hr and the rate of the fresh water was 400 gr/hr. Cyclization and distillation of the products was carried out as formerly described. The composition of all the products present in the aqueous effluent of the cyclization was again 71.7 mol% ε-caprolactam, 10.9 mol% 6ACA and 6ACAM and 17.4% oligomers. Again no N-methyl caprolactam was detected. After distillation 309 gr ε-caprolactam (2.73 mol) was recovered. The distillation residue (165 gr) should contain according to the mass-balance 1.38 mol ε-caprolactam and/or ε-caprolactam precursors.

Thus after 2 times recycle of the distillation residue an overall caprolactam yield of 91.2 mol% had been achieved based on the initial amount of M5FV. In the first cyclization pass where still some methanol was present 5.1 mol% of the unwanted by-product N-methyl caprolactam was formed. The approximated caprolactam yield per pass was 61.2% in the first pass, 66.5% in the second pass (first recycle of distillation residue) and 66.4% in the third pass (second recycle of distillation residue). Thus it was proven that after distillative caprolactam recovery the remaining distillation residue can successfully be recycled to the cyclization reactor resulting in a high overall caprolactam yield.

Example XV

The resulting effluent of Example XIII was continuously fed for 22 hours to the top of a steamstripper column at a rate of approx 550 gr/hr. Steam was generated in a reboiler of the column. To the column also 350 gr/hr of fresh water was fed. In the steamstripper column the liquid product stream was thus contacted with an upflowing stream of steam. The bottom temperature in the column was kept at 100°C. The liquid bottom stream which left the steamstripper at a rate of 742 gr/hr did not contain any detectable amount of methanol and $NH_3$. The concentration of ε-caprolactam and ε-caprolactam precursors in the liquid bottom stream was 22.1 wt% in water (1.33 mol/hr). After 22 hours 16.3 kg of this mixture was collected containing a total of 29.26 mol of ε-caprolactam and ε-caprolactam precursors (3.3 wt% 6ACA, 9.3 wt% 6ACAM, 6.9 wt% ε-caprolactam and 2.6 wt% oligomers).

This liquid mixture was fed continuously to the cyclization reactor as first described in Example XIV at a rate of approximately 500 gr/hr and a temperature of 300°C. The cyclization was carried out at 300°C, 10 MPa and at a residence time of approximately 30 minutes. After cooling and depressurizing the average composition of all the products present in the liquid aqueous stream amounted to 70.5 mol% ε-caprolactam, 10.8 mol% 6ACA(M) and 18.7 mol% oligomers. No N-methyl caprolactam was detected in this mixture.

In two consecutive semicontinuous distillation as described in Example XIV first water was removed from the product stream and secondly 2164 gr caprolactam (19.15 mol) was recovered from the product stream. The residue of the second distillation amounted to 1205 gr and according to the mass balance should contain a total of 10.13 mol of ε-caprolactam and ε-caprolactam precursors. The caprolactam yield in the first pass through the cyclization reactor was thus 65.4 mol%.

Comparison of this Example with Example XIV illustrate that by removing the methanol prior to performing the cyclization step the formation of N-methyl caprolactam can be avoided. Furthermore a higher yield to ε-caprolactam can be obtained for the illustrated situation, comprising a ones through cyclization and a subsequent second cyclization performed on the residue of the distillation after the first cyclization.

Example XVI

Example X was repeated with a feed having a composition of 24.4 wt% M5FV, 30.2 wt% $NH_3$, 6.8 wt% methanol and 38.6 wt% $H_2O$ was continuously fed to a two step reductive amination section as described in Example X. The flow rate corresponded with 191.6 gr/hr M5FV (1.33 mol/hr).

The mixture leaving the CSTR was continuously flashed at 0.11 MPa. Also 114 gr/hr $H_2O$ was continuously fed before the flasher to provide additional cooling. The product composition of the resulting liquid stream (approx. 550 gr/hr) consisted of 31 gr/hr methanol, 25 gr/hr ammonia, 330 gr/hr $H_2O$ and 164 gr/hr products of which 14.2 mol% 6ACA, 39.9 mol% 6ACAM, 33.9 mol% CAP and 12.0 mol% oligomers.

This mixture was continuously fed to a steamstripper column as described in Example XV. Also 350 gr/hr $H_2O$ is fed to the steamstripper column (bottom temperature is maintained at approximately 100°C). The remaining aqueous bottom stream having a rate of 742 gr/hr contained a total of 22.1 wt% of ε-caprolactam and ε-caprolactam precursors (1.33 mol/hr).

This mixture was continuously fed to a cyclization reactor as first described in Example XIV. Also 85 gr/hr (approx 0.715 mol/hr) of a recycle distillation residue (see below) and 314 gr/hr $H_2O$ were fed to the cyclization reactor. Thus overall 1141 gr/hr product mixture (21.8 wt% products) was fed to the cyclization reactor (249 gr/hr ε-caprolactam and ε-caprolactam precursors and 892 gr/hr $H_2O$).

The cyclization was carried out at 300°C, 10 MPa and at a residence time of approximately 30 minutes. After cooling and depressurizing, the effluent of the cyclization reactor was analyzed. The mixture consisted of 70.5 mol% ε-caprolactam, 10.8 mol% 6ACA(M) and 18.7 mol% oligomers.

This cyclization mixture was continuously fed to 2 consecutive vacuumdistillation columns. In the first column the solvent ($H_2O$) was removed. From the second column ε-caprolactam was recovered at a rate of 150 gr/hr (1.33 mol/hr).

The distillation residue obtained as the bottom stream in the second distillation (containing approximately a total of 0.715 mol/hr ε-caprolactam and ε-caprolactam precursors) was continuously recycled to the cyclization reactor (see above) at a rate of 85 gr/hr.

Thus virtually a 100% caprolactam yield could be obtained in a continuous reductive amination and cyclization process using a steamstripper to remove methanol before the cyclization and by recycling of the distillation residue after recovering part of the ε-caprolactam.

The above results were obtained 3 hours after the continuous process stabilized.

## Claims

1. Process for the preparation of ε-caprolactam starting from 5-formylvalerate acid, ester or amide (aldehyde compound) in which a reaction in the presence of ammonia and hydrogen and a subsequent cyclization of the reaction products thus formed (ε-caprolactam-precursors being 6-aminocaproate ester, 6-aminocaproic acid and/or 6-aminocaproamide) to ε-caprolactam is performed in the presence of water, **characterized in that** the following steps are performed

   (a) contacting the 5-formylvalerate acid, ester or amide with ammonia and water in the absence of hydrogen and/or a hydrogenation catalyst,
   (b) contacting the resulting mixture of step (a) with hydrogen in the presence of ammonia and a hydrogenation catalyst, in which the water content is higher than 10 wt.%,
   (c) heating the resulting mixture of step (b) at a temperature between 200 and 350°C in order to convert the reaction products of step (b) to ε-caprolactam.

2. Process according to claim 1, **characterized in that** the temperature in step (a) is between 20 and 100°C.

3. Process according to any one of claims 1-2, **characterized in that** the molar ratio of ammonia : aldehyde compound or aldehyde compound plus reaction products in step (a) is between 3:1 and 25:1.

4. Process according to any one of claims 1-3, **characterized in that** the water content in the reaction mixture in step (a) and (b) is between 15-60 wt.%.

5. Process according to any one of claims 1-4, **characterized in that** the hydrogenation conditions in step (b) are achieved by performing step (b) in the presence of a hydrogenation catalyst comprising one or more of the metals choosen from Group 8-10 of The Periodic System of Elements.

6. Process according to claim 5, **characterized in that** a ruthenium containing hydrogenation catalyst is used.

7. Process according to any one of claims 1-6, **characterized in that** the temperature in step (c) is between 285 and 330°C.

8. Process according any one of claims 1-7, **characterized in that** the starting compound is a 5-formylvalerate ester with the following general formula

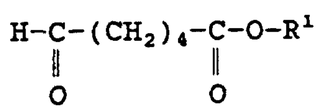

   in which $R^1$ is a 1 to 20 carbon atom alkyl group.

9. Process according to claim 8, **characterized in that** in the starting mixture of step (a) also the corresponding alkanol according to $R^1$-OH is present.

10. Process according to claim 8 or 9, **characterized in that** the content of the alkanol in step (c) is less than 1 wt%.

11. Process according to any one of claims 1-10, **characterized in that** ε-caprolactam is separated from the mixture obtained in step (c) and that the remaining mixture comprising unconverted ε-caprolactam precursors is recycled to step (c).

12. Process according to any one of claims 1-11, **characterized in that** the content of ε-caprolactam and ε-caprolactam precursors in step (c) is between 10 and 35 wt%.

13. Process according to claim 12, **characterized in that** the content of ε-caprolactam and ε-caprolactam precursors is more than 15 wt.%.

**14.** Process according to any one of claims 1-13, **characterized in that** the ammonia concentration in step (c) is below 5 wt.%.

## Patentansprüche

**1.** Verfahren zur Herstellung von ε-Caprolactam, ausgehend von 5-Formylvaleriansäure, -ester oder -amid (Aldehydverbindung), wobei eine Reaktion in Gegenwart von Ammoniak und Wasserstoff und eine anschließende Cyclisierung der so gebildeten Reaktionsprodukte (wobei ε-Caprolactam-Vorstufen, 6-Aminocapronsäureester, 6-Aminocapronsäure und/oder 6-Aminocaproamid sind) zu ε-Caprolactam in Gegenwart von Wasser ausgeführt wird, **dadurch gekennzeichnet, dass** die nachstehenden Schritte durchgeführt werden:

(a) In-Kontakt-Bringen der/des 5-Formylvaleriansäure, -esters oder -amids mit Ammoniak und Wasser in Abwesenheit von Wasserstoff und/oder einem Hydrierungskatalysator,

(b) In-Kontakt-Bringen des erhaltenen Gemisches von Schritt (a) mit Wasserstoff in Gegenwart von Ammoniak und einem Hydrierungskatalysator, wobei der Wassergehalt höher als 10 Gew.-% ist,

(c) Erhitzen des erhaltenen Gemisches von Schritt (b) auf eine Temperatur zwischen 200 und 350°C, um die Reaktionsprodukte von Schritt (b) zu ε-Caprolactam umzuwandeln.

**2.** Verfahren nach Anspruch 1, **dadurch gekennzeichnet, dass** die Temperatur in Schritt (a) zwischen 20 und 100°C liegt.

**3.** Verfahren nach einem der Ansprüche 1 bis 2, **dadurch gekennzeichnet, dass** das Molverhältnis von Ammoniak zu Aldehydverbindung oder Aldehydverbindung plus Reaktionsprodukte in Schritt (a) zwischen 3:1 und 25:1 liegt.

**4.** Verfahren nach einem der Ansprüche 1 bis 3, **dadurch gekennzeichnet, dass** der Wassergehalt in dem Reaktionsgemisch in Schritt (a) und (b) zwischen 15-60 Gew.-% liegt.

**5.** Verfahren nach einem der Ansprüche 1 bis 4, **dadurch gekennzeichnet, dass** die Hydrierungsbedingungen in Schritt (b) durch Ausführen von Schritt (b) in Gegenwart eines Hydrierungskatalysators, umfassend eines oder mehrere der Metalle, ausgewählt aus Gruppen 8-10 des Periodensystems der Elemente, erreicht werden.

**6.** Verfahren nach Anspruch 5, **dadurch gekennzeichnet, dass** ein Ruthenium enthaltender Hydrierungskatalysator verwendet wird.

**7.** Verfahren nach einem der Ansprüche 1 bis 6, **dadurch gekennzeichnet, dass** die Temperatur in Schritt (c) zwischen 285 und 330°C liegt.

**8.** Verfahren nach einem der Ansprüche 1 bis 7, **dadurch gekennzeichnet, dass** die Ausgangsverbindung ein 5-Formylvaleriansäureester mit der nachstehenden allgemeinen Formel

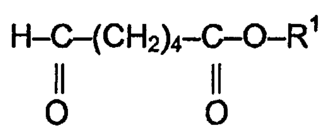

ist, worin $R^1$ eine Alkylgruppe mit 1 bis 20 Kohlenstoffatomen darstellt.

**9.** Verfahren nach Anspruch 8, **dadurch gekennzeichnet, dass** in dem Ausgangsgemisch von Schritt (a) auch das entsprechende Alkanol gemäß $R^1$-OH vorliegt.

**10.** Verfahren nach Anspruch 8 oder 9, **dadurch gekennzeichnet, dass** der Gehalt des Alkanols in Schritt (c) weniger als 1 Gew.-% ist.

**11.** Verfahren nach einem der Ansprüche 1 bis 10, **dadurch gekennzeichnet, dass** ε-Caprolactam von dem in Schritt (c) erhaltenen Gemisch getrennt wird und dass das verbleibende, nicht umgesetzte ε-Caprolactamvorstufen umfassende Gemisch zu Schritt (c) zurückgeführt wird.

**12.** Verfahren nach einem der Ansprüche 1 bis 11, **dadurch gekennzeichnet, dass** der Gehalt an ε-Caprolactam und ε-Caprolactamvorstufen in Schritt (c) zwischen 10 und 35 Gew.-% liegt.

**13.** Verfahren nach Anspruch 12, **dadurch gekennzeichnet, dass** der Gehalt an ε-Caprolactam und ε-Caprolactamvorstufen mehr als 15 Gew.-% ist.

**14.** Verfahren nach einem der Ansprüche 1 bis 13, **dadurch gekennzeichnet, dass** die Ammoniakkonzentration in Schritt (c) unterhalb von 5 Gew.-% liegt.

## Revendications

**1.** Procédé de préparation d'ε-caprolactame à partir d'acide, d'ester ou d'amide 5-formylvalérate (composé aldéhyde) dans lequel on effectue une réaction en présence d'ammoniac et d'hydrogène et une cyclisation consécutive des produits de réaction ainsi formés (les précurseurs d'ε-caprolactame étant l'ester 6-aminocaproate, l'acide 6-aminocaproïque et/ou le 6-aminocaproamide) en ε-caprolactame, en présence d'eau, **caractérisé en ce que** l'on met en oeuvre les étapes consistant :

(a) à mettre en contact l'acide, l'ester ou l'amide 5-formylvalérate avec l'ammoniac et l'eau en l'absence d'hydrogène et/ou de catalyseur d'hydrogénation,
(b) à mettre en contact le mélange aqueux obtenu de l'étape (a) avec de l'hydrogène en présence d'ammoniac et d'un catalyseur d'hydrogénation, la teneur en eau étant supérieure à 10% pds,
(c) à chauffer le mélange obtenu dans l'étape (b) à une température entre 200 et 350 °C afin de convertir les produits de réaction de l'étape (b) en ε-caprolactame.

**2.** Procédé selon la revendication 1, **caractérisé en ce que** la température dans l'étape (a) est comprise entre 20 et 100 °C.

**3.** Procédé selon l'une quelconque des revendications 1-2, **caractérisé en ce que** le rapport molaire ammoniac/composé aldéhyde ou composé aldéhyde plus produits de réaction dans l'étape (a) est compris entre 3/1 et 25/1.

**4.** Procédé selon l'une quelconque des revendications 1-3, **caractérisé en ce que** la teneur en eau dans le mélange réactionnel dans l'étape (a) et l'étape (b) est comprise entre 15 et 60 % pds.

**5.** Procédé selon l'une quelconque des revendications 1-4, **caractérisé en ce que** les conditions d'hydrogénation dans l'étape (b) sont obtenues en effectuant l'étape (b) en présence d'un catalyseur d'hydrogénation comprenant un ou plusieurs des métaux choisis dans le groupe 8-10 du tableau périodique des éléments.

**6.** Procédé selon la revendication 5, **caractérisé en ce que** l'on utilise un catalyseur d'hydrogénation contenant du ruthénium.

**7.** Procédé selon l'une quelconque des revendications 1-6, **caractérisé en ce que** la température dans l'étape (c) est comprise entre 285 et 330 °C.

**8.** Procédé selon l'une quelconque des revendications 1-7, **caractérisé en ce que** le composé de départ est un ester 5-formylvalérate répondant à la formule générale suivante:

$$\begin{array}{l} H-\underset{\underset{O}{\|}}{C}-(CH_2)_4-\underset{\underset{O}{\|}}{C}-O-R^1 \end{array} \qquad (1)$$

dans laquelle $R^1$ est un groupe alkyle contenant 1 à 20 atomes de carbone.

9. Procédé selon la revendication 8, **caractérisé en ce que** dans le mélange de départ de l'étape (a), l'alcanol correspondant à $R^1$-OH est également présent.

10. Procédé selon la revendication 8 ou 9, **caractérisé en ce que** la teneur en alcanol dans l'étape (c) est inférieure à 1 % pds.

11. Procédé selon l'une quelconque des revendications 1-10, **caractérisé en ce que** l'ε-caprolactame est séparé du mélange obtenu dans l'étape (c) et **en ce que** le mélange restant comprenant des précurseurs d'ε-caprolactame non convertis est recyclé dans l'étape (c).

12. Procédé selon l'une quelconque des revendications 1-11, **caractérisé en ce que** la teneur en ε-caprolactame et en précurseurs d'ε-caprolactame dans l'étape (c) est comprise entre 10 et 35 % pds.

13. Procédé selon la revendication 12, **caractérisé en ce que** la teneur en ε-caprolactame et en précurseurs d'ε-caprolactame est supérieure à 15 % pds.

14. Procédé selon l'une quelconque des revendications 1-13, **caractérisé en ce que** la concentration d'ammoniac dans l'étape (c) est inférieure à 5 % pds.

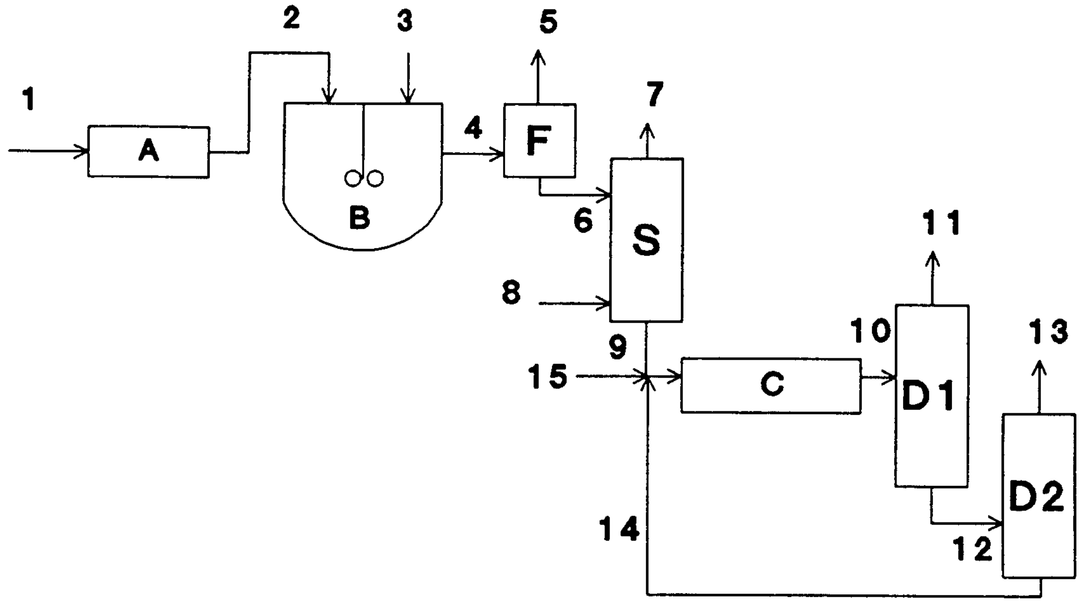
1
A
2
3
B
4
5
F
7
6
S
8
9
15
C
10
11
D1
13
D2
12
14